# EUROPEAN PATENT APPLICATION

(11) **EP 0 850 985 A1**
(43) Date of publication of application: **01.07.1998**
(21) Application number: 97122545.3
(22) Date of filing: 19.12.1997
(51) Int. Cl.: C08K 5/29, C08G 18/79, C08L 75/06

(54) **Improver for resistance of ester group-containing resin to microbe-caused deterioration, method for improving said resistance and resin composition having resistance to microbe-caused deterioration**

(30) Priority: 24.12.1996 JP 356319/96
(71) Applicant: NISSHINBO INDUSTRIES, INC., Chuo-ku, Tokyo 103 (JP)
(72) Inventor: Imashiro, Yasuo, Nisshinbo Industries Inc., Chuo-ku, Tokyo 103 (JP); Takahashi, Ikuo, Nisshinbo Industries Inc., Chuo-ku, Tokyo 103 (JP); Horie, Naofumi, Nisshinbo Industries Inc., Chuo-ku, Tokyo 103 (JP); Yamane, Takeshi, Nisshinbo Industries Inc., Chuo-ku, Tokyo 103 (JP); Suzuki, Shigekazu, Nisshinbo Industries Inc., Chuo-ku, Tokyo 103 (JP)
(74) Representative: Laufhütte, Dieter, Dr.-Ing.

(57) **Abstract**

The present invention provides:
an improver for resistance of ester group-containing resin to microbe-caused deterioration, which contains, as a main component, a carbodiimide compound represented by the general formula (1) R₁-NCN-R_{2,} (2) OCN-R₃-(NCN-R₄)ₙ-NCO, (3) R₅-NCN-R₆-(NCN-R₇)ₙ-NCN-R₈, or (4) R₉-R₁₀-(NCN-R₁₁)ₙ-R₁₂.
a method for improving the resistance of ester group-containing resin to microbe-caused deterioration, which comprises mixing an ester group-containing resin or reacting part of said resin with at least one of the carbodiimide compounds of the above general formulas (1) to (4), and
a resin composition having a resistance to microbe-caused deterioration, which is obtained by mixing an ester group-containing resin or reacting part of said resin with at least one of the carbodiimide compounds of the above general formulas (1) to (4).

The improver of the present invention, when added to an ester group-containing resin, can allow the resin to have an improved resistance to microbe-caused deterioration.

## Description

### Background of the Invention

### (1) Field of the Invention

The present invention relates to an improver for resistance of ester group-containing resin to deterioration caused by microbe such as fungi or the like; a method for improving said resistance; and a resin composition having a resistance to microbe-caused deterioration.

### (2) Description of the Prior Art

Generally when a film or a molded material both made of an ester group-containing resin is in use, fungi, actinomyces and/or bacteria adhere thereto; and these microbes decompose the resin, ingest the decomposition products as nutrition sources, and grow. As a result, the film or the molded article causes deformation, deterioration, decomposition, corrosion, etc., resulting in reduced strength, cracking, blistering, etc. and, importantly, shortened life.

In order to prevent the microbe-caused deterioration of resin product, various measures have been proposed heretofore. For example, there are disclosed, in Japanese Patent Application Laid-Open No. 59175/1991, (1) a method which comprises supporting, on a porous material such as zeolite, molecular sieve or the like, an antifungal agent having a antifungal property or the like, for example, an antifungal agent composed mainly of a metal (e.g. zinc, lead, tin or silver) or an organometal compound thereof, and adding the resulting material to a resin by melt kneading or the like; in Japanese Patent Application Laid-Open No. 175969/1990, (2) a method which comprises treating the surface of a resin with a compound having an antifungal property; and in Japanese Patent Application Laid-Open No. 173927/1990, (3) a method which comprises, using, as a raw material polyol in resin production, a polyol of particular structure having a antifungal property to allow the resulting resin to have a resistance to fungi-caused deterioration and consequently an extended life.

When an anti-fungal agent-supported inorganic material is melt-kneaded with, for example, a thermoplastic resin according to the above-mentioned method (1), the growth of fungi on the surface of the resin is suppressed by the action of the anti-fungal agent. However, in order to obtain a desirable effect, the concentration of the anti-fungal agent on the resin surface must be high, which necessitates addition of a large amount of the anti-fungal agent-supported inorganic material to the resin; as a result, the resin comes to contain the inorganic material in a large amount even in the interior (which need not have substantially any anti-fungal property) and the mechanical properties inherently possessed by the resin are significantly impaired.

Moreover, the anti-fungal agent oozes out from the inorganic material and even from the resin, impairing the appearance of the resin product and giving a bad influence to the environment; and the inorganic material as a carrier of the anti-fungal agent has poor compatibility with the resin, requiring a large energy and a long time for melt kneading.

When the surface of a resin is treated with an anti-fungal compound according to the method (2), the anti-fungal compound is easily detached from the resin by water washing, surface friction or the like; as a result, it is difficult to allow the resin to keep an anti-fungal property over a long period of time, the appearance of the resin is impaired, and the detachment of the anti-fungal compound gives a bad influence to the environment.

When a polyol of particular structure having an anti-fungal property is used as a raw material polyol according to the method (3), there are various problems. That is, the polyol is expensive and unavailable commercially; the resulting resin has properties greatly different from those of conventionally used resins; the polyol of particular structure has properties (e.g. reactivity, viscosity and OH value) different from those of conventionally used raw material polyols, and it has a big influence on the process for resin production; it is difficult to allow a conventional resin to have an anti-fungal property by a simple way; and the effect imparted by the method (3) is not satisfactory.

### Objects and Summary of the Invention

The objects of the present invention are to alleviate the above-mentioned problems of the prior art and to provide (a) an improver for resistance of ester group-containing resin to microbe-caused deterioration, which when added to an ester group-containing resin, can allow the resin to have an improved resistance to microbe-caused deterioration inexpensively and stably over a long period of time without impairing the mechanical properties inherently possessed by the resin or without causing the oozing out of the active component of the improver from the resin; (b) a method for improving said resistance of ester group-containing resin to microbe-caused deterioration; and (c) a resin composition having a resistance to microbe-caused deterioration.

According to the present invention, there is provided an improver for resistance of ester group-containing resin to microbe-caused deterioration, which contains, as a main component, a carbodiimide compound represented by the following general formula (1):

R₁-NCN-R₂ (1)

(wherein R₁ and R₂ may be the same or different and are each a residue of an aliphatic, alicyclic or aromatic monoisocyanate), or a carbodiimide compound represented by the following general formula (2):

OCN-R₃-(NCN-R₄)ₙ-NCO (2)

(wherein R₃ and R₄ may be the same or different and are each a residue of an aliphatic, alicyclic or aromatic diisocyanate, and n is an integer of 1-30), or a carbodiimide compound represented by the following general formula (3):

R₅-NCN-R₆-(NCN-R₇)ₙ-NCN-R₈ (3)

(wherein R₆ and R₇ may be the same or different and are each a residue of an aliphatic, alicyclic or aromatic diisocyanate; R₅ and R₈ may be the same or different and are each a residue of an aliphatic, alicyclic or aromatic monoisocyanate; and n is an integer of 1-30), or a carbodiimide compound represented by the following general formula (4):

R₉-R₁₀-(NCN-R₁₁)ₙ-R₁₂ (4)

(wherein R₁₀ and R₁₁ may be the same or different and are each a residue of an aliphatic, alicyclic or aromatic diisocyanate; R₉ and R₁₂ may be the same or different and are each a residue formed by a reaction of isocyanate group with an alcohol, an amine, a carboxylic acid or an acid anhydride; and n is an integer of 1-30).

According to the present invention, there is also provided a method for improving the resistance of ester group-containing resin to microbe-caused deterioration, which comprises mixing an ester group-containing resin or reacting part of said resin with at least one of the carbodiimide compounds of the general formulas (1) to (4) mentioned above.

According to the present invention, there is also provided a resin composition having a resistance to microbe-caused deterioration, which is obtained by mixing an ester group-containing resin or reacting part of said resin with at least one of the carbodiimide compounds of the general formulas (1) to (4) mentioned above.

### Detailed Description of the Invention

The present invention is hereinafter described in detail.

The "ester group-containing resin" to which the present invention is applied, includes, for example, a polyethylene terephthalate resin, a polybutylene terephthalate resin, a polyester polyurethane resin, an unsaturated polyester resin, a maleic acid resin, an alkyd resin, a polycarbonate resin and a polyester carbonate resin.

The "microbe-caused deterioration" mentioned in the present invention refers to a phenomenon that the ester bond present in molecules of resin is hydrolyzed by an esterase released from a microbe and, as a result, the resin gives rise to deformation, deterioration, decomposition, corrosion, etc.

The improver of the present invention for resistance of ester group-containing resin to microbe-caused deterioration contains, as a main component, a carbodiimide compound represented by one of the above-mentioned formulas. The carbodiimide compound can be produced by a carbodiimidization reaction (wherein an isocyanate is used as a raw material and a carbon dioxide-removing reaction takes place) or by dehydration and desulfurization of urea or thiourea.

The carbodiimidization reaction is conducted in the presence of an appropriate carbodiimidization catalyst. The carbodiimidization catalyst is preferably an organic phosphorus compound or an organometal compound represented by the general formula M-(OR)ₓ (wherein M is a metal such as Ti, Na, K, V, W, Hf, Zr, Pb, Mn, Ni, Ca, Ba or the like, R is a C₁₋₂₀ alkyl group or an aryl group, and X is a valence number of said metal). In view of the activity, a phospholene oxide is preferred as the organic phosphorus compound, and an alkoxide of Ti, Hf or Zr is preferred as the organometal compound.

The phospholene oxide can be exemplified by 3-methyl-1-phenyl-2-phospholene-1-oxide, 3-methyl-1-ethyl-2-phospholene-1-oxide, 1,3-dimethyl-2-phospholene-1-oxide, 1-phenyl-2-phospholene-1-oxide, 1-ethyl-2-phospholene-1-oxide, 1-methyl-2-phospholene-1-oxide and double bond isomers thereof. Of these, particularly preferred is 3-methyl-1-phenyl-2-phospholene-1-oxide which is easily available commercially.

The alkoxide of Ti, Hf or Zr is particularly preferably a tetraisopropyl, tetrabutyl or tetra-2-ethylhexyl alkoxide of Ti, Hf or Zr which is in industrial use as an ester condensation catalyst and is easily available.

The carbodiimidization reaction can be conducted by a conventional process. A raw material, for example, 4,4'-dicyclohexylmethane diisocyanate is stirred together with 0.1-10% by weight (a larger amount is possible when the economy is neglected), preferably 0.5-5% by weight, based on the diisocyanate, of the above-mentioned catalyst at 150-200°C in a solvent inert to the raw material or in the absence of any solvent, in an inert gas (e.g. nitrogen) current or with bubbling of the inert gas, whereby a carbodiimidization reaction (which gives rise to carbon dioxide removal) is allowed to proceed.

The rate of the carbodiimidization reaction differs depending upon the kind of diisocyanate used, the reaction temperature used, the catalyst amount used, etc. Since a high reaction rate makes it difficult to control polymerization, the carbodiimidization reaction is preferred to be conducted at an appropriate catalyst amount and an appropriate reaction temperature. For example, when an aliphatic diisocyanate is subjected to carbodiimidization, the catalyst amount is preferably 0.1-10% by weight, more preferably 0.5-5% by weight and the reaction temperature is preferably 120-200°C; when an aromatic diisocyanate is subjected to carbodiimidization, the catalyst amount is preferably 0.01-5% by weight, more preferably 0.05-1% by weight and the reaction temperature is preferably 50-180°C.

The carbodiimide compound produced as above has a carbodiimide group number of preferably 1-30, more preferably 1-15 because such a carbodiimide compound is low in melting point and viscosity and high in dispersibility in and miscibility with resin.

When, in the above reaction, a monoisocyanate(s) is (are) used as the raw material isocyanate, there can be obtained a carbodiimide compound represented by the following general formula (1):

R₁-NCN-R₂ (1)

wherein R₁ and R₂ may be the same or different and are each a residue formed by removing isocyanate group from an aliphatic, alicyclic or aromatic monoisocyanate (e.g. C₁₋₆ lower alkyl isocyanate, isomer thereof, cyclohexyl isocyanate, phenyl isocyanate or 2,6-diisopropylphenyl isocyanate).

When a diisocyanate(s) is (are) used as the raw material isocyanate, there can be obtained a carbodiimide compound represented by the following general formula (2):

OCN-R₃-(NCN-R₄)ₙ-NCO (2)

wherein R₃ and R₄ may be the same or different and are each a residue formed by removing isocyanate group from an aliphatic, alicyclic or aromatic diisocyanate (e.g. 4,4'-dicyclohexylmethan e diisocyanate, tetramethylxylylene diisocyanate, isophorone diisocyanate, 2,4,6-triisopropylphenyl diisocyanate, 4,4'-diphenylmethane diisocyanate, tolylene diisocyanate or hydrogenated tolylene diisocyanate), and n is an integer of 1-30. When two or more kinds of diisocyanates are used as the raw material isocyanate, the mode of polymerization may be any of random and block.

The carbodiimide compound used in the present invention may be a compound obtained by subjecting the above-obtained carbodiimide compound having terminal isocyanates [the compound of formula (2)] to terminal blocking with a compound(s) having a group reactive with said isocyanates, such as amine, carboxylic acid, acid anhydride, monoisocyanate or/and the like.

As the monoisocyanate used for terminal blocking of carbodiimide compound, there can be mentioned, for example, a monoisocyanate selected from aliphatic isocyanates (e.g. butyl isocyanate), alicyclic isocyanates (e.g. cyclohexyl isocyanate) and aromatic isocyanates (e.g. phenyl isocyanate and 2,6-diisopropylphenyl isocyanate), or a mixture thereof.

When the terminal isocyanates of the carbodiimide compound represented by the general formula (2) are blocked with a monoisocyanate(s), there can be obtained a carbodiimide compound represented by the following general formula (3):

R₅-NCN-R₆-(NCN-R₇)ₙ-NCN-R₈ (3)

wherein R₆ and R₇ may be the same or different and are each a residue formed by removing isocyanate group from an aliphatic, alicyclic or aromatic diisocyanate (e.g. 4,4'-dicyclohexylmethan e diisocyanate, tetramethylxylylene diisocyanate, isophorone diisocyanate, 2,4,6-triisopropylphenyl diisocyanate, 4,4'-diphenylmethane diisocyanate, tolylene diisocyanate or hydrogenated tolylene diisocyanate); R₅ and R₈ may be the same or different and are each a residue formed by removing isocyanate group from an aliphatic, alicyclic or aromatic monoisocyanate (e.g. C₁₋₆ lower alkyl isocyanate, isomer thereof, cyclohexyl isocyanate, phenyl isocyanate or 2,6-diisopropylphenyl isocyanate); and n is an integer of 1-30.

In the above terminal blocking of carbodiimide compound with monoisocyanate(s), mixing of diisocyanate(s) and monoisocyanate(s) and subsequent carbodiimidization is advantageous in order to obtain a carbodiimide compound of desired molecular weight because this approach can reduce the amount of the monocarbodiimide formed by condensation of monoisocyanate (s).

When, in the carbodiimide compound represented by the general formula (3), the molar ratio of diisocyanate(s) and monoisocyanate(s) is 1:2 to 30:2, preferably 4:2 to 19:2. When the molar ratio is 3:2, the number of carbodiimide in molecule, i.e. n is 4; and when the molar ratio is 19:2, n is 20.

When the terminal isocyanates of the carbodiimide compound represented by the general formula (2) are blocked with a monofunctional compound(s) having a group reactive with isocyanate group, such as alcohol, amine, carboxylic acid or acid anhydride, there can be obtained a carbodiimide compound represented by the following general formula (4):

R₉-R₁₀-(NCN-R₁₁)ₙ-R₁₂ (4)

wherein R₁₀ and R₁₁ may be the same or different and are each a residue formed by removing isocyanate group from an aliphatic, alicyclic or aromatic diisocyanate (e.g. 4,4'-dicyclohexylmethane diisocyanate, tetramethylxylylene diisocyanate, isophorone diisocyanate, 2,4,6-triisopropylphenyl diisocyanate, 4,4'-diphenylmethane diisocyanate, tolylene diisocyanate or hydrogenated tolylene diisocyanate); R₉ and R₁₂ may be the same or different and are each a residue formed by a reaction of isocyanate group with an alcohol, an amine, a carboxylic acid or an acid anhydride; and n is an integer of 1-30.

In the reaction for obtaining any of the carbodiimide compounds represented by the general formula (1) to (4), the reaction time differs depending upon the reaction temperature, the kind and amount of the catalyst, etc. For example, when 4,4-dicyclohexylmethane diisocyanate (HMDI) (a raw material) is reacted in the presence of 0.5% by weight, based on the diisocyanate, of 3-methyl-1-phenyl-2-phospholene-1-oxide in the absence of any solvent at 180°C, there can be obtained, in about 10 hours, an HMDI-derived carbodiimide compound of polymerization degree (n) of 3 having isocyanate terminals.

Also when isophorone diisocyanate (IPDI) (a raw material) is reacted, the same applies. When 2,4,6-triisopropyl benzene diisocyanate (a raw material) is reacted in the presence of 0.3% by weight, based on the diisocyanate, of 3-methyl-1-phenyl-2-phospholene-1-oxide in the absence of any solvent at 120°C, there can be obtained, in about 6 hours, a carbodiimide compound of polymerization degree (n) of 5 having isocyanate terminals, derived from 2,4,6-triisopropylbenzene diisocyanate.

The proceeding of the above reaction can be confirmed by examining the infrared absorption spectrum of isocyanate group at 2,258 cm⁻¹, or by conducting titrimetry.

When the terminal isocyanate groups of the carbodiimide compound represented by the general formula (2) are blocked with a monofunctional compound(s) having a group reactive with isocyanate group, such as alcohol, amine, carboxylic acid or/and acid anhydride, the reaction between the terminal isocyanate groups and the monofunctional compound(s) may be conducted before carbodiimidization or after carbodiimidization has proceeded to a certain polymerization degree [in this case, the monofunctional compound(s) is (are) added in an amount equivalent to the amount of remaining isocyanate groups]. However, when the monofunctional compound(s) has (have) a low boiling point(s) and vaporizes (vaporize) during carbodiimidization, the above reaction is preferably conducted before carbodiimidization.

The present improver for resistance of ester group-containing resin to microbe-caused deterioration contains the above-mentioned carbodiimide compound as a main component. The improver is specifically a mixture of the carbodiimide compound and a base material (such as silver ion-supported zeolite). The present improver therefore contains 100% by weight, preferably about 30% by weight or more of the carbodiimide compound.

The ratio of the present improver (for resistance of ester group-containing resin to microbe-caused deterioration) added to the ester group-containing resin is 0.01-5% by weight, preferably 0.3-1% by weight in terms of weight % of carbodiimide compound. When the ratio is lower than 0.01% by weight, the amount of carbodiimide compound to resin is too small and no sufficient effect is obtained. When the ratio is higher than 5% by weight, the carbodiimide compound acts as a plasticizer to the ester group-containing resin and gives adverse effects on resin properties.

The present method for improving the resistance of ester group-containing resin to microbe-caused deterioration comprises, in the first mode, mixing an ester group-containing resin with at least one of the above carbodiimide compounds. There is no restriction as to the method of mixing, and an appropriate mixing method can be used. For example, pellets of an ester group-containing resin are mixed with the carbodiimide compound by dry blending, and the resulting mixture is melt-kneaded using an extruder or an injection molding machine generally at 160-220°C when the resin is a urethane resin, or at 250-300°C when the resin is a polyethylene terephthalate (PET), or at 230-280°C when the resin is a polybutylene terephthalate (PBT).

When the ester group-containing resin is dissolved or dispersed in a solvent or water or can be handled as a liquid, the mixing of the resin and the carbodiimide compound can be conducted using an ordinary mechanical stirrer. In that case, when the carbodiimide compound has no good miscibility with the solvent or water owing to the former's viscosity or low compatibility with the solvent or water, emulsification under heating may be conducted, or a surfactant, a solvent or the like may be used as necessary.

When the carbodiimide compound is reacted with the ester group-containing resin, it can be conducted, for example, by adding the carbodiimide compound having isocyanate terminals, at the time of resin synthesis to react the terminal isocyanate groups of the carbodiimide compound with the hydroxyl group, amino group or carboxyl group of resin material to form a urethane bond, a urea bond or an amide bond, respectively.

The thus-obtained resin composition of the present invention having a resistance to microbe-caused deterioration undergoes little deterioration caused by microbe even in soil or water without impairing the properties inherently possessed by the resin and, therefore, can keep the shape and strength over a long period of time.

The fungi to which the present invention is applicable, can be any fungi as long as they attack ester group-containing resins. They include, for example, Aspergillus, Eurotium, Penicillium, Fusarium, Trichoderma, Pestalotia, Alternaria, Epicoccum, Cladosporium, Ulocladium, Paecilomyces, Acremonium, Nigrospora, Rhizopus, Aureobasidium, Gliocladium, Chaetomium, Myrothecium, Mucor, Phoma, baker's yeast, Scolecobasidium, Actinomycetes, Bacillus, Colon Bacillus, Pseudomonas and Staphyloccus.

### Examples

The present invention is hereinafter described in more detail.

The abbreviations appearing in Tables 1-6 of Synthesis Examples indicate the followings.

### Isocyanates

- CHI:: Cyclohexyl isocyanate
- DII:: 2,6-Diisopropylphenyl isocyanate
- HMDI:: 4,4-Dicyclohexylmethane diisocyanate
- IPDI:: Isophorone diisocyanate
- TMXDI:: Tetramethylxylylene diisocyanate
- TIDI:: 2,4,6-Triisopropylphenyl diisocyanate
- TDI:: a mixture of 2,4-Tolylene diisocyanate and 2,6-tolylene diisocyanate
- MDI:: 4,4'-Diphenylmethane diisocyanate
- TODI:: Tolidine diisocyanate
- XDI:: Xylylene diisocyanate
- H6XDI:: Hydrogenated xylylene diisocyanate
- HDI:: Hexamethylene diisocyanate
- NDI:: Naphthalene diisocyanate

### Blocking agents

- CHI:: Cyclohexyl isocyanate
- n-Bu:: n-Butyl isocyanate
- DII:: 2,6-Diisopropylphenyl isocyanate
- Ph:: Phenyl isocyanate
- CHA:: Cyclohexylamine
- CHOH:: Cyclohexanol
- PEG3:: Polyethylene glycol monomethyl ether (number of ethylene groups = 3)
- PEG8:: Polyethylene glycol monomethyl ether (number of ethylene groups = 8)
- n-BuA:: n-Butylamine
- DBA:: Dibutylamine
- DCA:: Dicyclohexylamine

### Synthesis of carbodiimide compounds

### (Synthesis Examples 1-2) Synthesis of monocarbodiimides

To 100 g of CHI was added 2 g of 3-methyl-1-phenyl-2-phospholene-1-oxide (hereinafter abbreviated to carbodiimidization catalyst). The mixture was reacted at 170°C for 6 hours with stirring at 200 rpm using a mechanical stirrer, to obtain a CHI-derived carbodiimide compound (polymerization degree = 1) of Synthesis Example 1.

A monocarbodiimide of Synthesis Example 2 was obtained in the same manner as in the above Synthesis Example 1 except that the catalyst amount and the reaction temperature were changed as shown in Table 1.

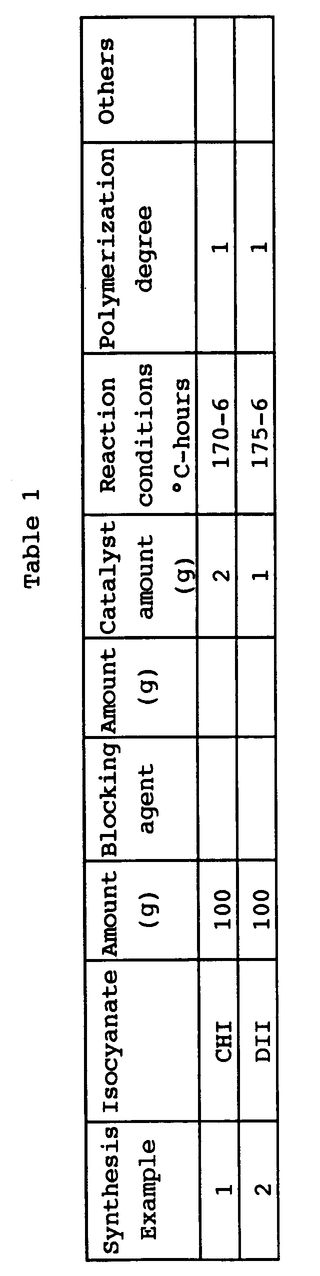

### (Synthesis Examples 3-16) Synthesis of NCO-terminated carbodiimides

To 300 g of HMDI was added 1.5 g of the carbodiimidization catalyst. The mixture was subjected to a condensation reaction at 185°C for 10 hours in a nitrogen current with stirring at 200 rpm using a mechanical stirrer, to obtain a HMDI-derived carbodiimide compound (polymerization degree = 5) of Synthesis Example 3.

Isocyanate-terminated carbodiimides of Synthesis Examples 4-16 were obtained in the same manner as in the above Synthesis Example 3 except that the diisocyanate kind, the catalyst amount and the reaction temperature and time were changed as shown in Table 2. Incidentally, in Synthesis Examples 11-16, each reaction was conducted in a Perclene solvent (20% of the amount of raw material diisocyanate) and the resulting slurry was dried by spray drying to obtain a carbodiimide compound.

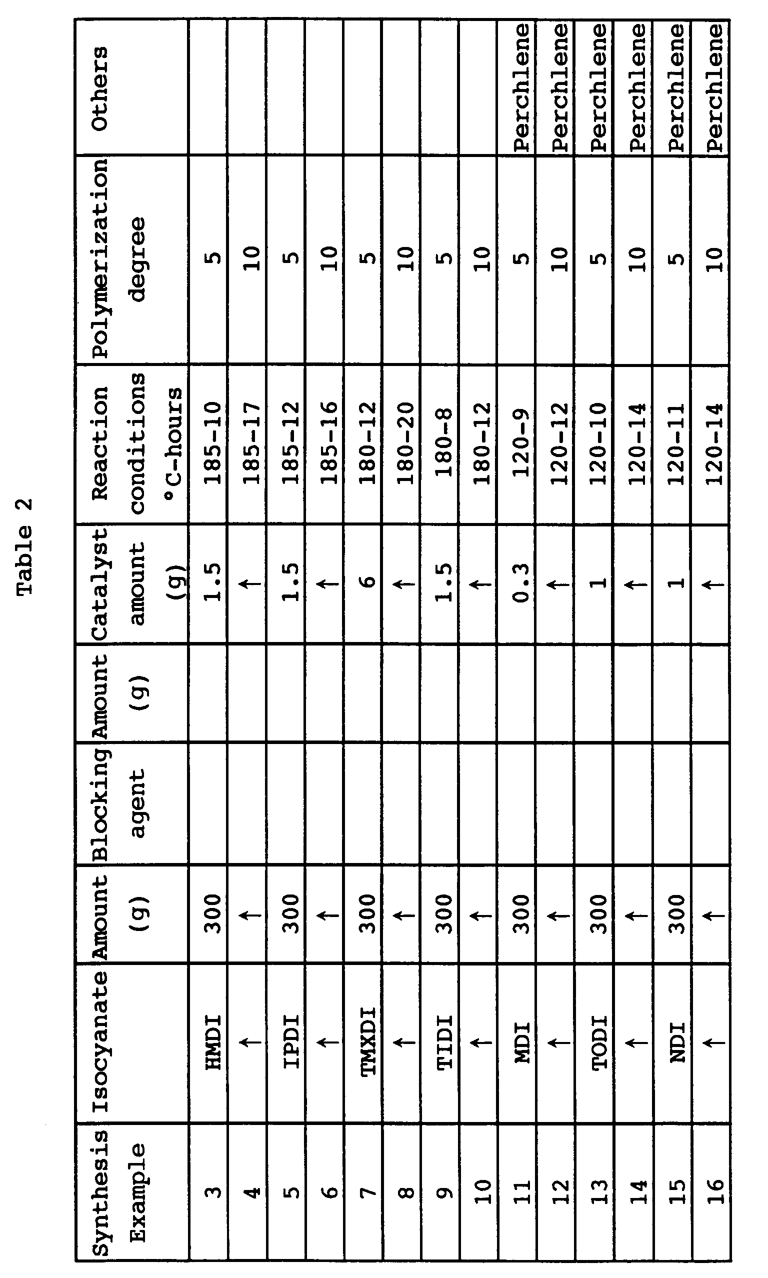

### (Synthesis Examples 17-34) Synthesis of terminal-blocked carbodiimides

To 105 g of HMDI was added 25 g of cyclohexyl isocyanate (a monoisocyanate as blocking agent). Thereto was added 1.3 g of the carbodiimidization catalyst. The mixture was reacted at 185°C for 14 hours with stirring at 200 rpm using a mechanical stirrer, to obtain a carbodiimide compound (polymerization degree = 5) of Synthesis Example 17.

Terminal-blocked carbodiimides of Synthesis Examples 18-34 were obtained in the same manner as in the above Synthesis Example 17 except that the diisocyanate kind and amount, the blocking agent kind and amount, the catalyst amount and the reaction temperature and time were changed as shown in Table 3. Incidentally, in Synthesis Examples 27-34, each reaction was conducted in a Perclene solvent (20% of the amount of raw material diisocyanate) and the resulting slurry was dried by spray drying to obtain a carbodiimide compound.

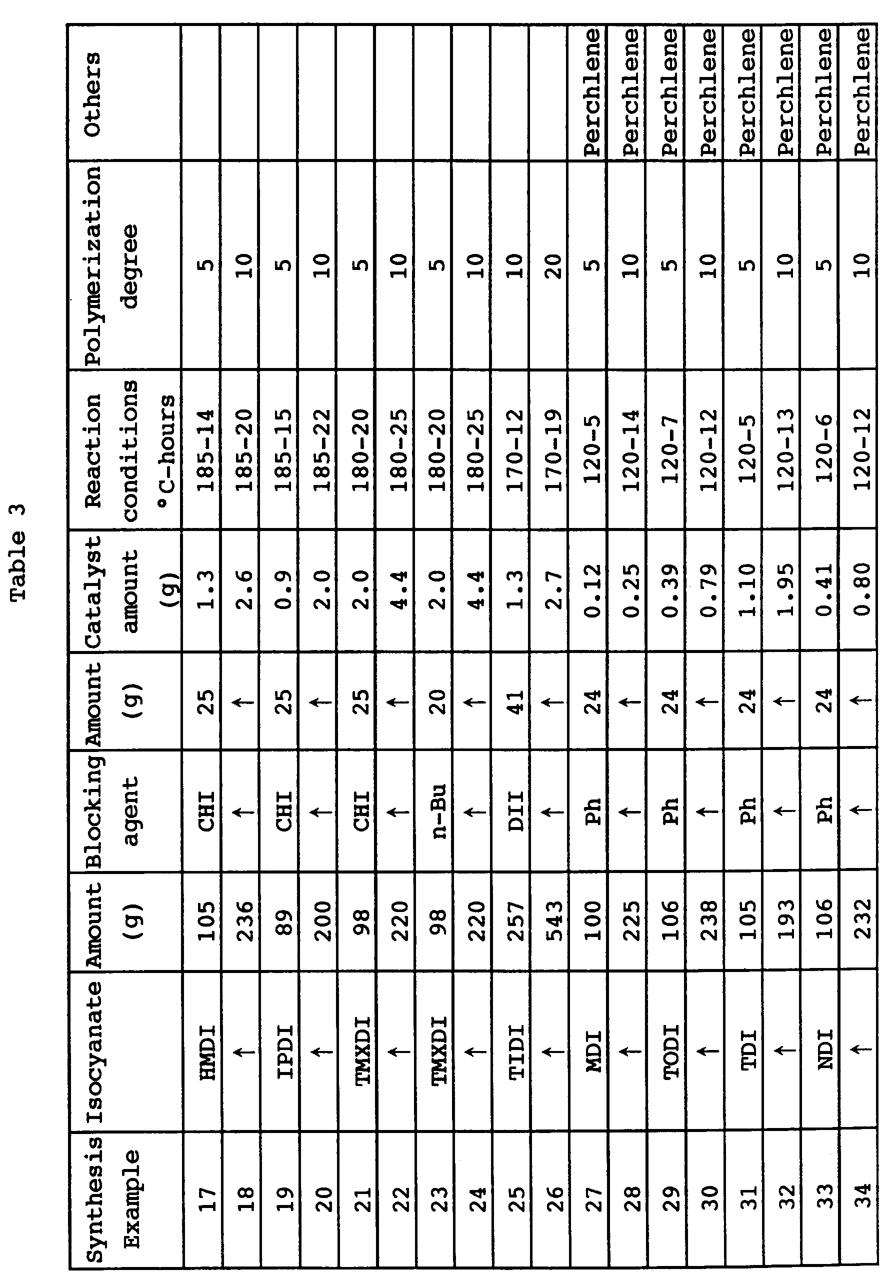

### (Synthesis Examples 35-80) Synthesis of terminal-blocked carbodiimides

To 236 g of HMDI was added 20 g of cyclohexylamine, and the mixture was stirred at 100°C for 1 hour with bubbling nitrogen. Thereto was added 2.4 g of the carbodiimidization catalyst, and the mixture was reacted at 185°C for 23 hours, to obtain a carbodiimide compound (polymerization degree = 10) derived from HMDI and having a urea bond at each terminal, of Synthesis Example 35.

Syntheses were conducted in the same manner as in the above Synthesis Example 35 except that the diisocyanate kind and amount, the catalyst amount, the reaction temperature and time and the kind and amount of blocking agent were changed as shown in Table 4, whereby carbodiimide compounds having, in the molecule, urea bond, urethane bond, imide bond or amide bond, of Synthesis Examples 36-80 were obtained. Incidentally, in Synthesis Examples 62-80, each reaction was conducted in a Perclene solvent (20% of the amount of raw material diisocyanate) and the resulting slurry was dried by spray drying to obtain a carbodiimide compound.

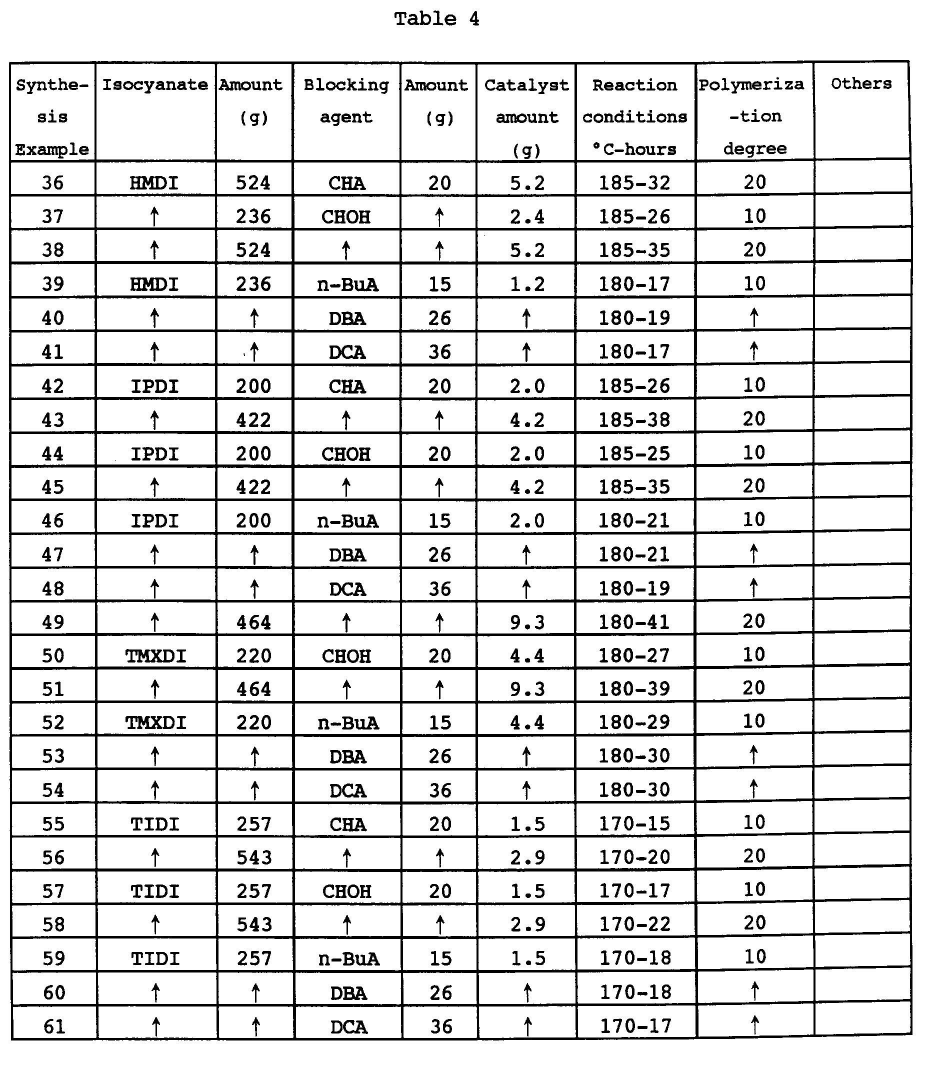

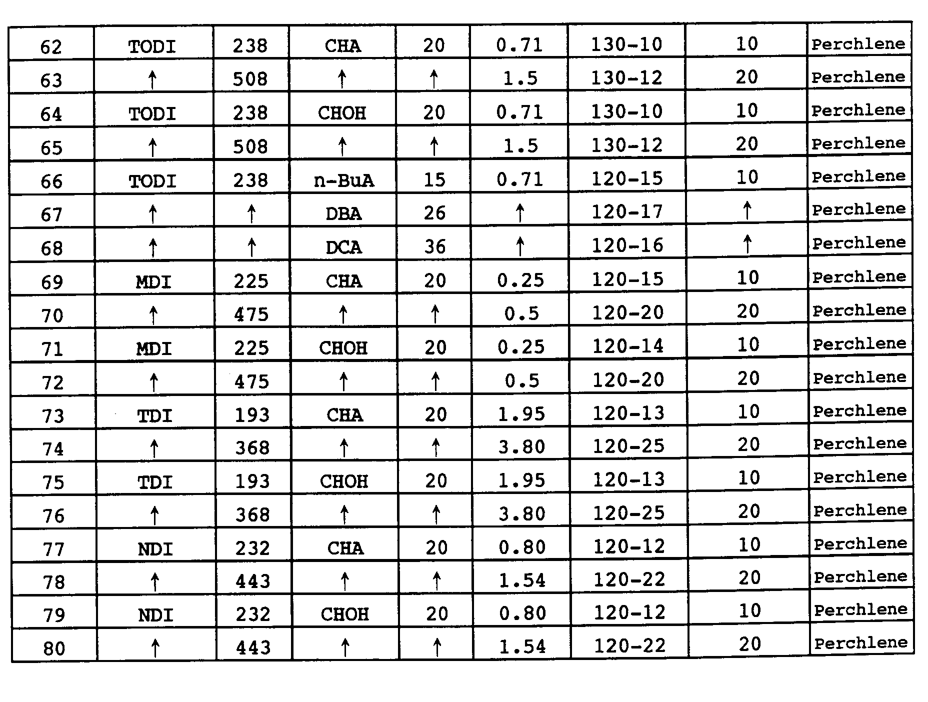

### (Synthesis Examples 81-86) Synthesis of terminal-blocked carbodiimides

A carbodiimide compound of Synthesis Example 3 was produced. The compound was reacted with 94 g of a polyethylene glycol monomethyl ether (number of ethylene groups = 3) (this ether is abbreviated to PEG3) at 130°C for 3 hours, to obtain a terminal isocyanate-blocked carbodiimide of Synthesis Example 81.

Terminal-blocked carbodiimides of Synthesis Examples 82-86 were obtained in the same manner as in the above Synthesis Example 81 except that the diisocyanate kind and amount, the catalyst amount, the blocking agent kind and amount, the reaction time were changed as shown in Table 5. Incidentally, when the catalyst kind was 3-methyl-1-phenyl-2-phospholene-1-oxide (an organic phosphorus compound), it was indicated as P in the catalyst amount column of Table 5; when the catalyst kind was tetrabutyl titanate (an organometal compound), it was indicated as T in the same column.

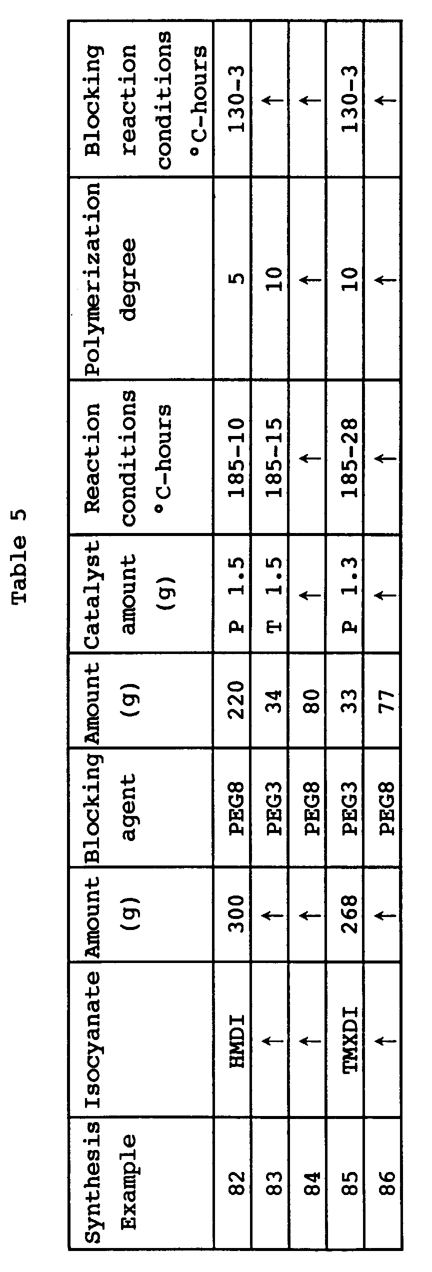

### (Synthesis Examples 87-94) Synthesis of terminal-blocked carbodiimides

192 g of TDI was added 33 g of PEG3, and the mixture was stirred at 60°C for 1 hour at 200 rpm using a mechanical stirrer. Thereto was added the carbodiimidization catalyst, and the mixture was reacted at 80°C for 6 hours to obtain a carbodiimide (polymerization degree = 10) of Synthesis Example 87.

Carbodiimides of Synthesis Examples 88-94 were obtained in the same manner as in the above Synthesis Example 87 except that the diisocyanate kind and amount, the blocking agent kind and amount, the catalyst amount and the reaction temperature and time were changed as shown in Table 6.

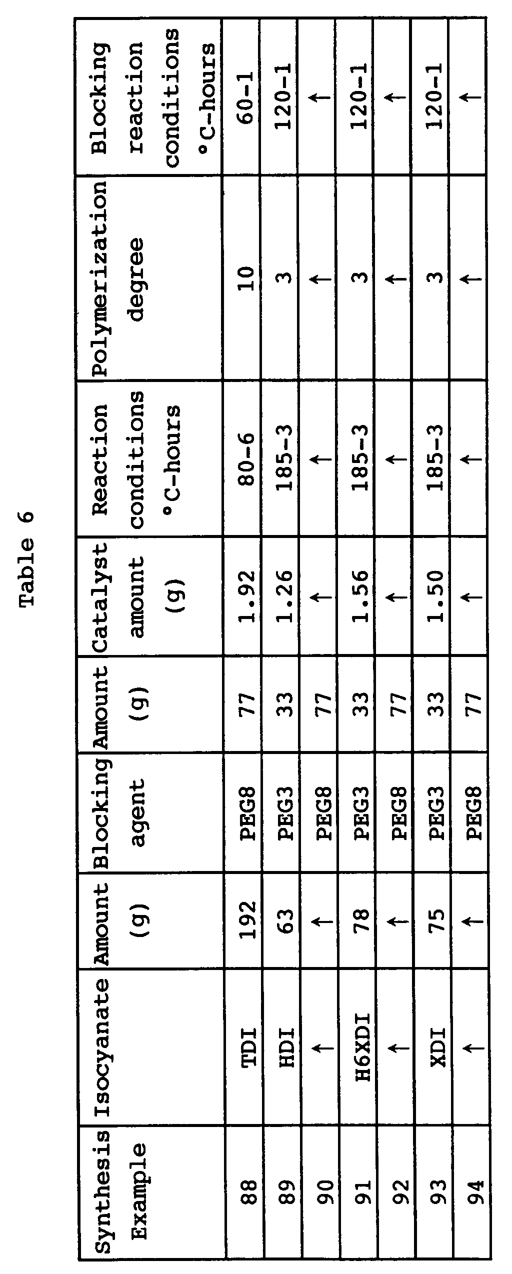

### Example 1

The following materials were reacted at 130°C for 1 hour using a mechanical stirrer.
o 11.17 kg of an adipic acid-based polyester polyol (molecular weight = 2,113)
o 7.5 kg or 15.0 kg of one of the carbodiimide compounds obtained in the above Synthesis Examples (0.5% or 1% relative to an urethane resin to be formed)
o 1.99 kg of MDI
Thereto was added 1.22 kg of MDI, and a reaction was conducted for 15 minutes to obtain an isocyanate-terminated prepolymer. To the prepolymer was added 624 g of butanediol to obtain various carbodiimide-containing urethane resins.

Each urethane resin was subjected to melt extrusion using a single-screw extruder having a T die, to form a film of about 200 micron. The film was punched into dumbbell No. 4 specimens. The specimens were subjected to a soil burial test. The results were evaluated by tensile strength retention (%) when the original tensile strength was taken as 100. The standard of evaluation was as follows.
- ⓞ:: Tensile strength retention is 80% or more.
- ○:: Tensile strength retention is 50% or more.
- △:: Tensile strength retention is 10% or more.
- X:: Tensile strength retention is less than 10%.

### Example 2

Preparation of specimens and a soil burial test were conducted in the same manner as in Example 1 except that there was used, as the ester group-containing resin, a polybutylene terephthalate (PBT) elastomer (Grilux EH-70, a product of Dainippon Ink & Chemicals, Inc.).

### Example 3

Preparation of specimens and a soil burial test were conducted in the same manner as in Example 1 except that there was used, as the ester group-containing resin, a polyethylene terephthalate (PET) resin (EFG-7, a product of Kanebo, Ltd.).

### Comparative Example

Preparation of specimens and a soil burial test were conducted in the same manner as in Examples 1-3 except that no carbodiimide compound was added.

The results of soil burial test are shown in Tables 7-11.

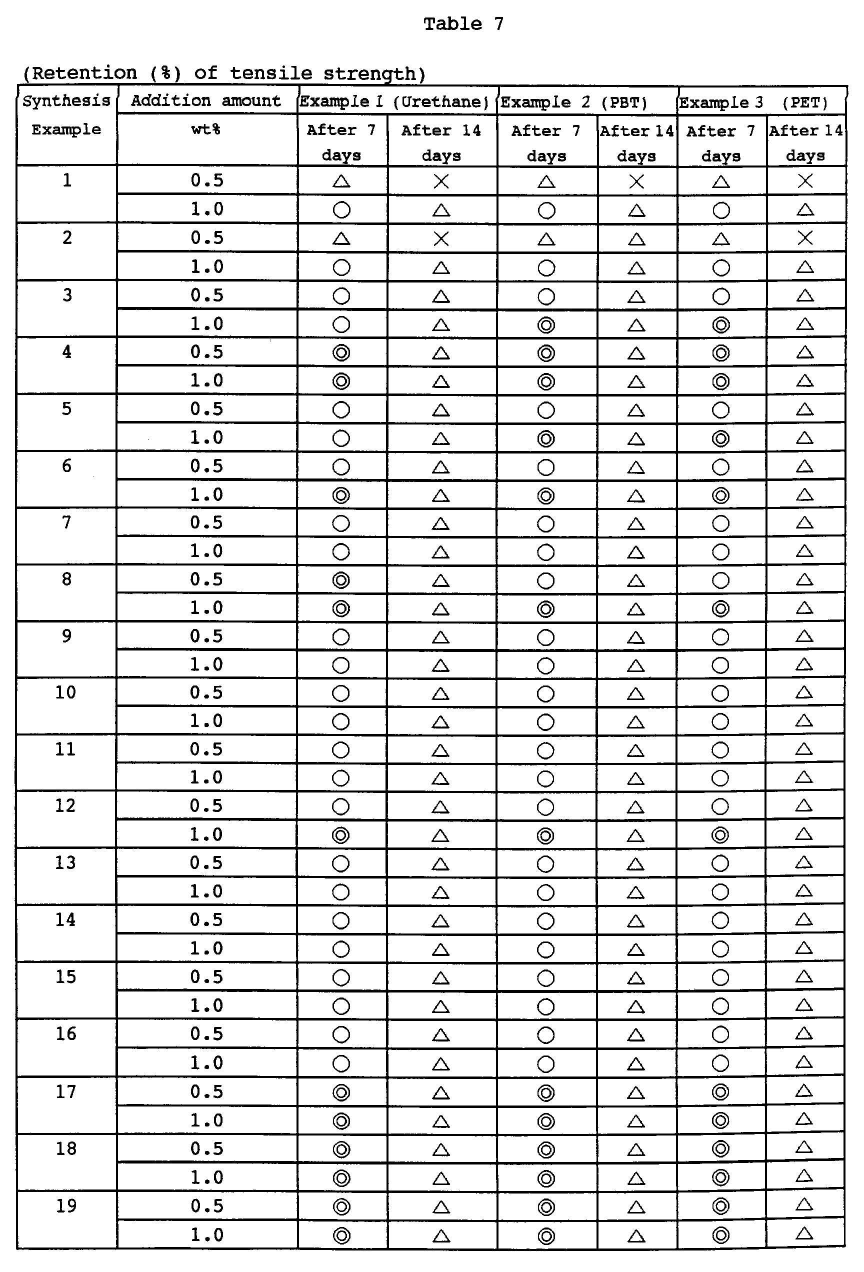

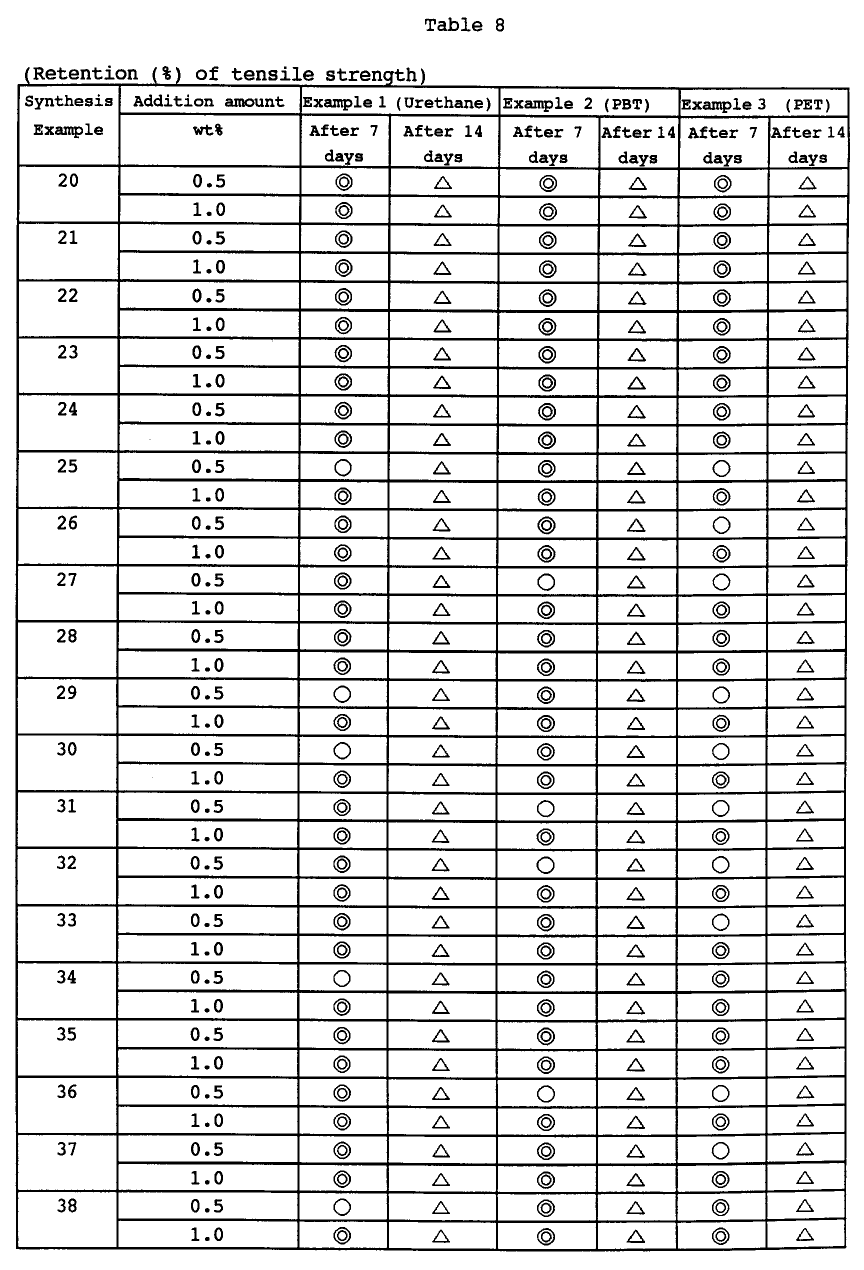

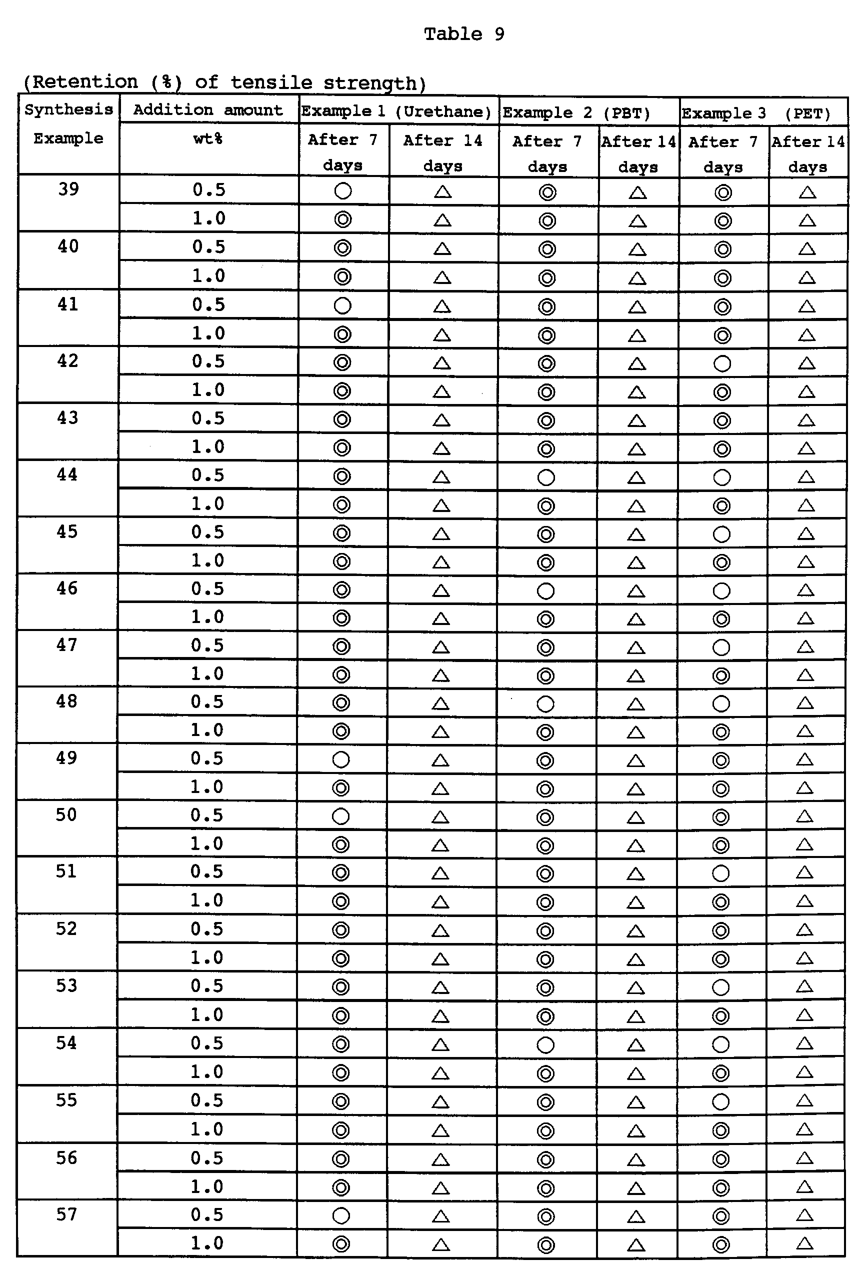

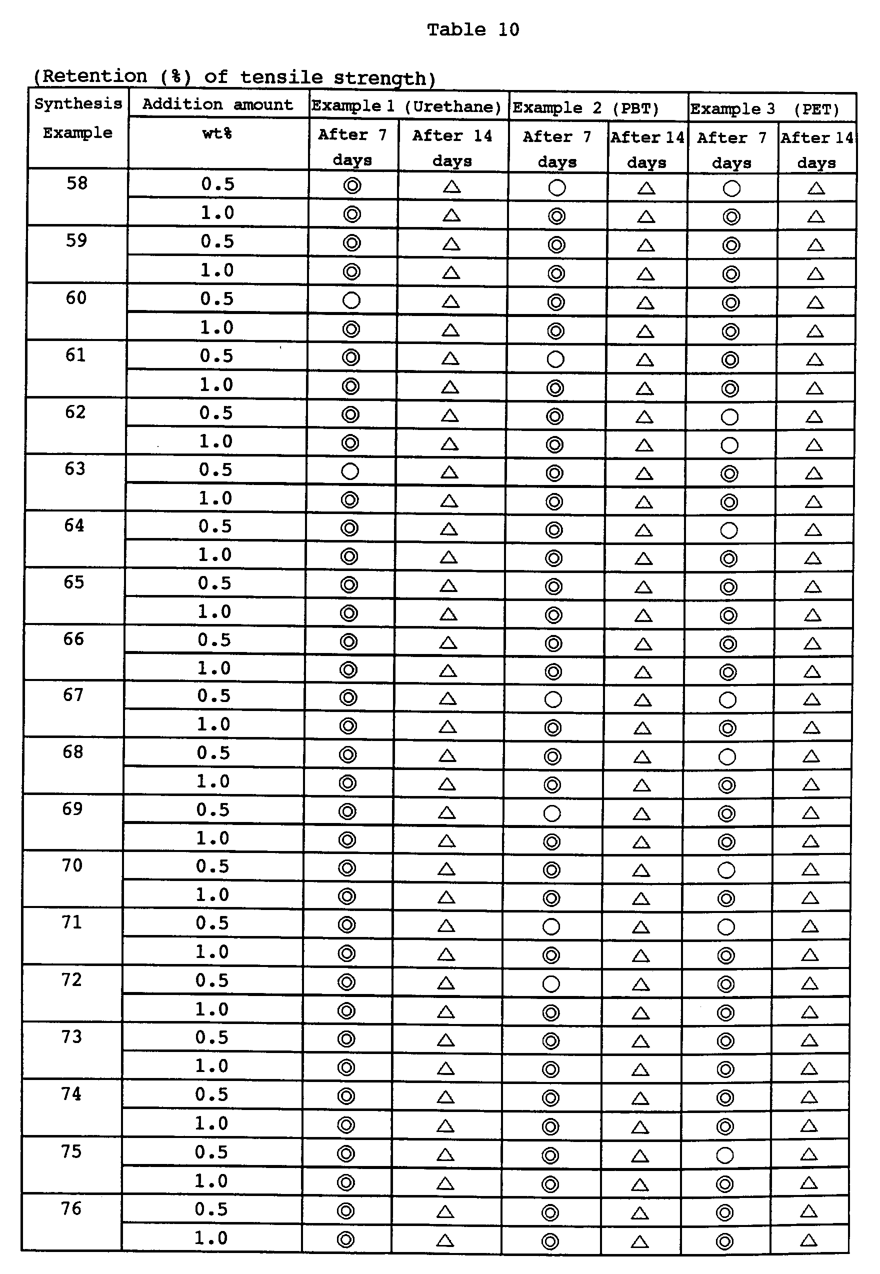

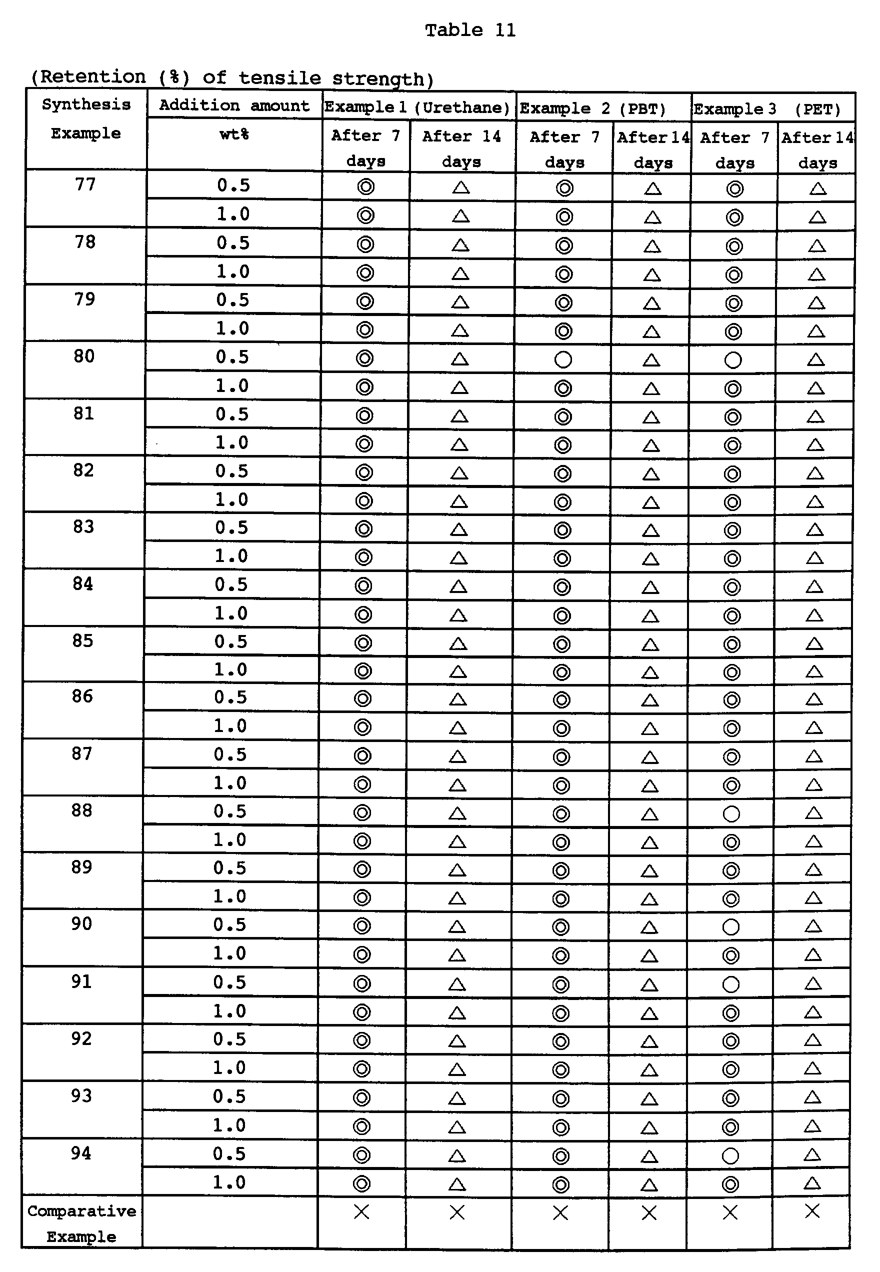

For information, the main fungi found in the specimens after soil burial test are shown below.

Pastalotia adusta, Curvularia geniculata, Penicillium nigricans, Arthrinium sacchari, Ulocladium atrum, Drechslera australiensis, Cladosporium sphaerospermum, Fusarium oxysporum, Aspergillus niger, Aspergillus flavus, Penicillium citrinium, Chaetomium globosum, Fusarium moniliforme.

According to the present invention, there can be provided a resin which, when buried in microbes-present soil over a long period of time, undergoes little attack by microbes and show inherent properties stably over a long period of time.

## Claims

1. An improver for resistance of ester group-containing resin to microbe-caused deterioration, which contains, as a main component, a carbodiimide compound represented by the following general formula (1):
R₁-NCN-R₂ (1)
wherein R₁ and R₂ may be the same or different and are each a residue of an aliphatic, alicyclic or aromatic monoisocyanate.

2. An improver for resistance of ester group-containing resin to microbe-caused deterioration, which contains, as a main component, a carbodiimide compound represented by the following general formula (2):
OCN-R₃-(NCN-R₄)ₙ-NCO (2)
wherein R₃ and R₄ may be the same or different and are each a residue of an aliphatic, alicyclic or aromatic diisocyanate, and n is an integer of 1-30.

3. An improver for resistance of ester group-containing resin to microbe-caused deterioration, which contains, as a main component, a carbodiimide compound represented by the following general formula (3):
R₅-NCN-R₆-(NCN-R₇)ₙ-NCN-R₈ (3)
wherein R₆ and R₇ may be the same or different and are each a residue of an aliphatic, alicyclic or aromatic diisocyanate; R₅ and R₈ may be the same or different and are each a residue of an aliphatic, alicyclic or aromatic monoisocyanate; and n is an integer of 1-30.

4. An improver for resistance of ester group-containing resin to microbe-caused deterioration, which contains, as a main component, a carbodiimide compound represented by the following general formula (4):
R₉-R₁₀-(NCN-R₁₁)ₙ-R₁₂ (4)
wherein R₁₀ and R₁₁ may be the same or different and are each a residue of an aliphatic, alicyclic or aromatic diisocyanate; R₉ and R₁₂ may be the same or different and are each a residue formed by a reaction of isocyanate group with an alcohol, an amine, a carboxylic acid or an acid anhydride; and n is an integer of 1-30.

5. A method for improving the resistance of ester group-containing resin to microbe-caused deterioration, which comprises mixing an ester group-containing resin or reacting part of said resin with at least one of the carbodiimide compounds of the general formulas (1) to (4) mentioned in Claims 1-4.

6. A resin composition having a resistance to microbe-caused deterioration, which is obtained by mixing an ester group-containing resin or reacting part of said resin with at least one of the carbodiimide compounds of the general formulas (1) to (4) mentioned in Claims 1-4.
